# EUROPÄISCHE PATENTANMELDUNG

(11) **EP 3 915 605 A1**
(43) Veröffentlichungstag der Anmeldung: **01.12.2021**
(21) Anmeldenummer: 20176822.3
(22) Anmeldetag: 27.05.2020
(51) Int. Cl.: A61M 1/00

(54) **FÖRDERVORRICHTUNG FÜR KÖRPERFLÜSSIGKEIT**

(71) Anmelder: Medela Holding AG, 6340 Baar (CH)
(72) Erfinder: Ehlert, Hilmar, 6052 Hergiswil Nidwalden (CH)
(74) Vertreter: Grünecker Patent- und Rechtsanwälte PartG mbB

(57) **Zusammenfassung**

Die vorliegende Erfindung betrifft eine Fördervorrichtung für Körperflüssigkeit mit einem Katheterschlauch (2), der ein in den Körper einzuführendes distales Ende (4) und ein proximales Ende (6) hat. Zur Schaffung einer Fördervorrichtung, die sich nicht mehr mit koagulierenden Substanzen zusetzen kann, wird mit der vorliegenden Erfindung ein Hohlkörper (14) vorgeschlagen, der längsverschieblich und/oder drehbar in dem Katheterschlauch (2) angeordnet ist, sich von dem distalen Ende (4) zumindest bis zu dem proximalen Ende (6) erstreckt und über seine Länge einen sich in Erstreckungsrichtung des Katheterschlauches erstreckenden Transportkanal ausbildet.

## Beschreibung

Die vorliegende Erfindung betrifft eine Fördervorrichtung für Körperflüssigkeit mit einem Katheterschlauch, der ein in dem Körper einzuführendes distales Ende und ein proximales Ende hat.

Eine derartige Fördervorrichtung ist beispielsweise aus WO 2017/152125 A2 bekannt. Die aus diesem Stand der Technik vorbekannte Lösung wurde in Ansehung des Problems vorgeschlagen, dass insbesondere aus dem Brustbereich und im Rahmen eines chirurgischen Eingriffs am Herzen eine Körperflüssigkeit absaugende Fördervorrichtung in Form eines Katheterschlauches verstopfen kann. Die aus WO 2017/152125 A2 vorbekannte Lösung schlägt dazu vor, das proximale Ende des in den Körper eingeführten Katheterschlauches an mehrere parallel verlegte Schläuche anzuschließen, so dass beispielsweise ein Schlauch als Drainageschlauch für das Abziehen der Körperflüssigkeit genutzt werden kann, wohingegen ein anderer Schlauch durch Einleiten eines Spülmediums, beispielsweise über einen dritten Schlauch, gespült werden kann. Dadurch soll das Verstopfen der Fördereinrichtung durch koagulierende Bluttropfen bzw. andere Körpersubstanzen vermieden werden.

Die vorbekannte Lösung kann aber nicht das Verstopfen des in dem Körper aufgenommenen Katheterschlauches verhindern. Die nach WO 2017/152125 A2 vorgeschlagenen Maßnahmen greifen lediglich in demjenigen Teil der Fördervorrichtung, die außerhalb des Körpers liegt.

Der vorliegenden Erfindung liegt das Problem zugrunde, eine Fördervorrichtung der eingangs genannten Art anzugeben, die in verbesserter Weise dem Verstopfen des Katheterschlauches durch koagulierende Substanzen aus dem Körper entgegenwirkt.

Zur Lösung dieses Problems wird mit der vorliegenden Erfindung eine Fördervorrichtung mit den Merkmalen von Anspruch 1 angegeben.

Die erfindungsgemäße Fördervorrichtung hat einen Hohlkörper, der längsverschieblich und/oder drehbar insbesondere frei drehbar in dem Katheterschlauch angeordnet ist und sich von dem distalen Ende zumindest bis zu dem proximalen Ende des Katheterschlauches ggf. auch darüber hinaus erstreckt. Der Hohlkörper durchsetzt dementsprechend den gesamten Katheterschlauch. Der Hohlkörper ist dabei bevorzugt als länglicher Biegekörper oder -welle ausgebildet, d. h. in Längsrichtung ohne Beeinträchtigung der Drehbarkeit des Hohlkörpers in dem Katheterschlauch biegbar. Die Biegbarkeit in diesem Sinne beträgt bevorzugt zumindest 90°. Gemäß der Erfindung ist der Hohlkörper in Erstreckungsrichtung des Katheterschlauches hohl. Diese Höhlung erstreckt sich in Erstreckungsrichtung des Katheterschlauches und bildet hierin einen Transportkanal aus. So kann trotz des Vorhandenseins des Hohlkörpers ein Transport von Fluid, beispielsweise durch Saugen an dem proximalen Ende durch den Katheterschlauch erfolgen. Der Hohlkörper kann besagte Höhlung in seinem Inneren ausbilden. Die äußere Abmessung des Hohlkörpers kann in etwa dem Innendurchmesser des Katheterschlauches entsprechen. In diesem Falle wird die Höhlung üblicherweise entlang der Mittellängsachse des Hohlkörpers ausgebildet.

Der Hohlkörper kann für sich elastische und rückstellende Eigenschaften haben. Er ist bevorzugt durch eine Vielzahl von Windungen gebildet oder weist diskrete Abstandselemente auf, die sich radial in Bezug auf die Längserstreckung des Hohlkörpers erstrecken. Bei einem von außen wirkenden Druck sorgt der Hohlkörper unter Beibehaltung des Transportkanals für eine gewisse Freistellung des Katheterschlauches, sodass dieser nicht knicken und verlegt werden kann. Damit wird die heute regelmäßig geübte Praxis obsolet, sicherheitshalber mehrere nach außen führende Katheterschläuche beispielsweise für die Drainage des Perikards zu legen, um auch bei einer Verlegung eines der Schläuche eine ausreichende Drainage sicherzustellen. Auch kann der Katheterschlauch dünnwandiger als zuvor ausgebildet werden.

Der Hohlkörper kann auch den Transport von korpuskulären Bestandteilen wie beispielsweise Koagulat durch den Katheterschlauches verbessern. Abhängig von den Strömungsbedingungen innerhalb des Katheterschlauches kann ein solches Koagulat durch die Teilverlegung des Katheterschlauches an diesem geschert und verkleinert werden.

Der Hohlkörper ist bevorzugt mit einem helixförmigen Schabsegment ausgebildet, welches sich radial von einer Drehachse des Hohlkörpers erstreckt. Bei einer Drehung und/oder Längsverschiebung des Hohlkörpers in dem Katheterschlauch zwischen dem Hohlkörper und der inneren Mantelfläche des Katheterschlauches anhaftende koagulierte Blutstropfen mechanisch aufbrechen, so dass eine an dem Katheterschlauch angeschlossene Saugquelle die in dem Katheterschlauch vorgesehene Flüssigkeit aus diesem besser absaugen kann. Es können auch mehrere Schabsegmente hintereinander angeordnet sein.

Solche Schabsegmente sind üblicherweise über die gesamte axiale Länge des Hohlkörpers verteilt vorgesehen, so dass die koagulierenden Substanzen im Grunde über die gesamte Länge des Katheterschlauches abgeschabt werden können.

Die Vermittlung der Bewegung des Hohlkörpers, beispielsweise eines Drehmoments auf den Hohlkörper kann auf verschiedene Weise erfolgen. So kann der Hohlkörper berührungsfrei und beispielsweise magnetisch angetrieben werden, indem ein magnetisches Antriebselement an der Außenumfangsfläche des Katheterschlauches vorgesehen und an entsprechender Stelle, bevorzugt im Bereich des proximalen Endes des Hohlkörpers dieses magnetische Antriebselement mit dem Hohlkörper verbunden wird. Bevorzugt ragt der Hohlkörper über das freie Ende des Katheterschlauches hinaus und ist dort antriebsmäßig mit einem Antrieb verbunden.

Es ist zu bevorzugen, den Hohlkörper durch eine Helix auszubilden, so dass die Rotation des Hohlkörpers in dem Katheterschlauch auch zum Fördern des Inhalts des Katheterschlauches nach Art einer archimedischen Schraube führt. Dabei kann die Helix einen Außendurchmesser haben, der in etwa dem Innendurchmesser des Katheterschlauches entspricht, so dass die Helix bei einer Rotation vollumfänglich an dem Katheterschlauch schabt.

Wie oben dargelegt hat der Hohlkörper bevorzugt über seine gesamte Länge sich radial erstreckende Schabsegmente. Diese Schabsegmente ragen radial von einer Drehachse und/oder Längsverschiebung des Hohlkörpers beispielsweise mit einem axialen Abstand zueinander ab, so dass eine Drehung und/oder Längsverschiebung des Hohlkörpers dazu führt, dass an der Innenfläche des Katheterschlauches anhaftendes koaguliertes Material abgeschabt wird bzw. dass das Anhaften von Beginn an unterbunden oder verunmöglicht wird. Die Schabsegmente können mit einem axialen Abstand zueinander vorgesehen sein, da sich die von den Schabsegmenten bewirkte Scherung von Anhaftungen an dem Katheterschlauch über eine gewisse axiale bzw. umfängliche Länge fortsetzt. Ist der Hohlkörper durch eine Helix gebildet, werden die besagten Schabsegmente jeweils kontinuierlich hintereinander zusammenhängend durch die Außenumfangsfläche der Helix gebildet, die bei der Drehung des Hohlkörpers um 360° jeweils einmal an einer bestimmten Position der Innenumfangsfläche des Katheterschlauches vorbeistreichen und somit Anhaftungen an der Oberfläche des Katheterschlauches abschaben.

Im Hinblick auf eine verbesserte Förderung insbesondere von korpuskulären Bestandteilen in Körperflüssigkeit beim Eintritt in den Katheterschlauch wird gemäß einer bevorzugten Weiterbildung der vorliegenden Erfindung vorgeschlagen, den Katheterschlauch im Bereich seines distalen Endes an seiner Mantelfläche mit zumindest einer Öffnung, bevorzugt mit mehreren an dem Umfang verteilten Öffnungen zu versehen. Der Hohlkörper hat bei dieser bevorzugten Ausgestaltung zumindest eine mit der Öffnung zusammenwirkende Schabkante, so dass bei Drehung des Hohlkörpers in der Öffnung liegende korpuskuläre oder zelluläre Bestandteile der abzusaugenden Körperflüssigkeit zerkleinert werden können. Solche Ausgestaltungen sind grundsätzlich aus WO 2005/084562 A2 bzw. US 6,264,667 B1, EP 470 888 B1 oder US 5,569,178 B bekannt. Im Gegensatz zu diesem Stand der Technik hat die erfindungsgemäße Fördervorrichtung bevorzugt sich über die gesamte Länge des Hohlkörpers verteilende Schabsegmente, die radial in Bezug auf die Drehachse des Hohlkörpers abgehen, jedenfalls aber einen Hohlkörper, der regelmäßig die Schabkante relativ zu der Öffnung bewegt. Während bei dem zuvor erwähnten Stand der Technik entsprechende Ausgestaltungen Gewebe oder Zellen vor dem Eintritt in den Katheterschlauch zerkleinern sollen, kann die erfindungsgemäße Fördervorrichtung zusätzlich durch die über die Länge des Hohlkörpers vorzugsweise gleichmäßig verteilten Schabsegmente das Anhaften von Partikeln an der Innenumfangsfläche des Katheterschlauches verhindern oder solche anhaftenden Partikel aufbrechen. Die erfindungsgemäße Fördervorrichtung ermöglicht den Transport von Festkörpern, welche unter dem bloßen Einfluss von Vakuum nicht bewegt werden können. Bei der vorliegenden Lösung werden diese mittels des bevorzugt mit zumindest einem helixförmigen Schabsegment ausgeführten Hohlkörper mechanisch herausgefördert.

Der Katheterschlauch ist üblicherweise endseitig offen, so dass Flüssigkeit axial in den Katheterschlauch eingesaugt und auch axial aus dem Katheterschlauch abgegeben werden kann. Gleichwohl hat der Katheterschlauch nach einer bevorzugten Weiterbildung der vorliegenden Erfindung an seinem distalen Ende einen axialen Anschlag für den Hohlkörper. Dieser axiale Anschlag umgibt üblicherweise die Einlassöffnung in den Katheterschlauch, die mit der axial durchgehenden Höhlung des Hohlkörpers fluchtet.

Die Vermittlung einer rotatorischen und/oder translatorischen Bewegung des Hohlkörpers erfolgt bevorzugt durch ein Antriebselement, in welches der Hohlkörper einbringbar, ggf. auch durch welches der Hohlkörper hindurchführbar und mit dem der Hohlkörper koppelbar ist. Die Kopplung erfolgt dabei bevorzugt zwangsläufig beim Einbringen des Hohlkörpers in das Antriebselement beispielsweise durch eine elastische Verformung des Antriebselementes und/oder des Hohlkörpers. Das Einbringen erfolgt bevorzugt durch axiales Einschieben des Hohlkörpers in das Antriebselement.

Gemäß einer bevorzugten Weiterbildung der vorliegenden Erfindung hat die Fördervorrichtung eine Kopplungseinheit mit einem Kopplungsgehäuse. Dieses Kopplungsgehäuse weist einen Katheterschlauchanschluss und einen Saugleitungsanschluss auf. Dabei ist der Katheteranschluss zum fluiddichten Anschluss des Katheterschlauches angepasst ausgebildet. Der Katheteranschluss kann beispielsweise ein Luer-Anschluss, ein anderer Bajonett-Anschluss oder ein offenes Schlauchende ohne speziellen Anschluss sein. Der Katheteranschluss kann auch durch einen Anschlusszapfen ausgebildet sein, auf dem der Katheter mit seinem proximalen Ende aufgebschoben werden kann. Der Saugleitungsanschluss, der in entsprechender Weise ausgebildet sein kann, kommuniziert mit einer Saugleitung, die zu einem Auffangbehälter für die abgesaugte Körperflüssigkeit führt. Bei der hier diskutierten Weiterbildung ist in dem Kopplungsgehäuse ein Durchflusskanal vorgesehen. Dieser Durchflusskanal kommuniziert mit dem Katheterschlauchanschluss und dem Saugleitungsanschluss. Der Durchflusskanal ist üblicherweise zwischen diesen beiden Anschlüssen angeordnet.

Drehbar an dem Kopplungsgehäuse ist bevorzugt ein Antriebselement zum drehenden Antrieb des Hohlkörpers gelagert. So schafft die Kopplungseinheit einerseits eine fluiddichte Verbindung zwischen dem Katheterschlauch und der Saugleitung und ermöglicht andererseits die Übertragung einer Antriebskraft oder eines Drehmoments von dem Antriebselement auf den Hohlkörper. Dieser Hohlkörper überragt innerhalb der Kopplungseinheit den Katheterschlauch und kann dementsprechend unmittelbar mechanisch angetrieben werden. Das Antriebselement kann dabei fest oder lediglich antriebsmäßig mit dem Hohlkörper verbunden sein. Das Antriebselement ist bevorzugt in Form eines Hülsenantriebselementes ausgebildet, das mit einer verdrehfest mit dem Hohlkörper verbundenen Hülse antriebsmäßig gekoppelt ist und diese in sich aufnehmen und/oder von außen antreiben kann.

Das Antriebselement kann eine Kurvenbahn ausbilden oder mit einer solchen verbunden sein, die mit einer ortsfesten Gegenfläche, beispielsweise einem Nocken zusammenwirkt, beispielsweise unter Vorspannung dagegen anliegt, sodass eine Drehbewegung des Antriebselementes zwangsläufig auch zu einer axialen Verschiebung desselben und damit zu einer zyklischen translatorischen Bewegung des Hohlkörpers in dem Katheterschlauch führt.

Die vorliegende Erfindung lässt sich von der Vorstellung leiten, dass zwar der Katheterschlauch auf die gewünschte Länge im Rahmen der chirurgischen Versorgung eines Menschen abgeschnitten werden kann, nicht aber der Hohlkörper abgeschnitten werden muss. So hat der Saugleitungsanschluss einen zum Durchführen des Hohlkörpers angepasst ausgebildeten Innendurchmesser. Üblicherweise durchragt der Hohlkörper nach der antriebsmäßigen Kopplung des Hohlkörpers mit dem Antriebselement sowohl die Kopplungseinheit wie auch den Saugleitungsanschluss sowie eine Teillänge der Saugleitung. So sind dementsprechend auch die Innendurchmesser der Anschlüsse für den Katheter und die Saugleistung bzw. das Antriebselement bzw. eine Antriebshülse, die den Hohlkörper in sich aufnimmt und koppelt, zum Durchführen des Hohlkörpers angepasst ausgebildet.

Der Durchflusskanal ist gemäß einer bevorzugten Weiterbildung zumindest teilweise durch eine Hülse gebildet, die drehbar und längsverschieblich in dem Kopplungsgehäuse gelagert ist. Diese Hülse ist lösbar mit dem Hohlkörper verbindbar, also koppelbar im Sinne der vorliegenden Erfindung. Die Hülse kann das Antriebselement ausbilden oder lediglich antriebsmäßig damit verbunden sein. Die Kopplung kann beispielsweise durch eine die Hülse durchsetzende und gegen den Hohlkörper wirkende Madenschraube oder dergleichen erfolgen. Alternativ kann die Hülse auch radial in Grenzen elastisch beweglich sein, so dass der Hohlkörper gegen einen gewissen Widerstand durch die Hülse hindurchgeschoben werden kann, gleichwohl aber eine im Grunde feste, insbesondere verdrehfeste Verbindung zwischen dem Hohlkörper und der Hülse bewirkt wird und zwar derart, dass beim Antrieb der Hülse diese die Antriebskraft bzw. das Drehmoment auf den Hohlkörper vermittelt, so dass dieser sich innerhalb des Katheterschlauches dreht und/oder darin längs verschoben, insbesondere zyklisch hin und her bewegt wird. Der Hohlkörper ist danach bevorzugt kraft- und/oder formschlüssig mit der Hülse verbunden. Die Hülse ist üblicherweise abgedichtet in dem Kopplungsgehäuse aufgenommen, so dass die abgesaugte Flüssigkeit ohne Leckage von dem Katheterschlauch in die Saugleitung überführt werden kann.

Im montierten Zustand ist die Hülse üblicherweise vollständig innerhalb des Kopplungsgehäuses aufgenommen, wobei zumindest einer der Schlauchanschlüsse lösbar an dem Kopplungsgehäuse befestigt sein und die Hülse innerhalb des Kopplungsgehäuses abdecken kann. Die fluiddichte Verbindung kann dabei durch eine Dichtung bewirkt werden, die zwischen dem lösbaren Schlauchanschluss und dem Kopplungsgehäuse vorgesehen ist. Auch ist die Hülse lösbar antriebsmäßig mit dem Antriebselement verbindbar. Dieses Antriebselement kann beispielsweise ein mechanisches Antriebselement, beispielsweise eines Getriebes, oder ein magnetisch mit der Hülse gekoppeltes Antriebselement sein. Das Antriebselement kann auf eine Antriebshülse einwirken, die die zuvor erwähnte Hülse in sich aufnimmt und umfänglich umgibt. Diese Antriebshülse kann mit Magneten oder einer Verzahnung versehen sein.

Gemäß einer bevorzugten Weiterbildung ist eine Spülleitung vorgesehen, die mit dem Katheterschlauch und/oder der Saugleitung bzw. dem Durchflusskanal kommuniziert. Diese Spülleitung kann beispielsweise unmittelbar an den Katheterschlauch nahe seines distalen Endes angeschlossen sein, um den Transport durch Katheterschlauch durch zyklisches Spülen zu verbessern. Die Spülleitung kann aber auch erst außerhalb des Körpers angeschlossen sein. Auch dort kann der Katheterschlauch und/oder die Kopplungseinheit bzw. Saugleitung einen Spülanschluss für die Spülleitung aufweisen. Der Spülanschluss kann an dem Kopplungsgehäuse, bevorzugt auf der Katheterseite der Antriebseinheit angebracht sein, um auch die Antriebseinheit spülen zu können. Üblicherweise wird das über die Spülleitung bzw. den Spülanschluss eingebrachte Spülfluid über die Saugleitung abgeführt und somit die Fördervorrichtung zumindest teilweise gespült.

Die erfindungsgemäße Fördervorrichtung hat bevorzugt einen Antrieb, der antriebsmäßig mit dem Antriebselement koppelbar ist. Diese Kopplung kann beispielsweise durch eine Biegewelle oder eine andere Drehmomentübertragung erfolgen. Der Antrieb ist im Hinblick auf hygienische Anforderungen regelmäßig als separate Einheit zu der Kopplungseinheit vorgesehen und mit der Kopplungseinheit lediglich antriebsmäßig gekoppelt. So kann die Kopplungseinheit auch als Verbrauchsteil hergestellt und nach der Benutzung entsorgt werden. Dem Antrieb ist eine Antriebssteuerung zugeordnet, die derart ausgebildet ist, dass der Hohlkörper über den Antrieb kontinuierlich und/oder reversierend und/oder zyklisch drehend antreibbar ist. So kann der Hohlkörper auf verschiedene Weise angetrieben werden, insbesondere um an der Umfangsfläche des Katheterschlauches anhaftendes Koagulat abzuschaben oder Klots (Festkörper) rein mechanisch durch den Katheter zu fördern.

Der Antrieb des Hohlkörpers kann bevorzugt abhängig von dem Grad der Verstopfung des Katheterschlauches erfolgen. Hierzu hat die Antriebssteuerung in der Regel eine Leistungssteuerung, beispielsweise eine Drehmomenterkennung. Das von dieser erkannte Drehmoment geht beispielsweise in die Steuerung des Antriebs ein. Wird beispielsweise aufgrund einer erheblichen Koagulation eine für die gewünschte Relativbewegung des Hohlkörpers innerhalb des Katheterschlauches relativ große Leistungsaufnahme auf Seiten des Antriebs notwendig, erfolgt ein zeitlich länger dauernder Antrieb des Hohlkörpers im Vergleich zu einer unkritischen Drehmoment- oder anderen Leistungserkennung des Antriebs, die auf einen allerhöchstens geringfügig verstopften Katheterschlauch hinweist.

Gemäß einer bevorzugten Weiterbildung der vorliegenden Erfindung ist die Antriebssteuerung für den Antrieb in baulicher Einheit mit einer Saugsteuerung einer Saugpumpe verwirklicht. Diese Saugpumpe ist in an sich bekannter Weise einem Auffangbehälter zugeordnet, der mit der Saugleitung verbindbar ist. Solche Auffangbehälter mit zugeordneter Saugpumpe und Saugsteuerung sind für das Absaugen von Körperflüssigkeiten allgemein bekannt, beispielsweise aus WO 2018/054833 A2. Durch das einheitliche Ausbilden von Antriebssteuerung und Saugsteuerung kann der apparative Aufwand zur Herstellung der Fördervorrichtung verringert werden. Darüber hinaus kann das Absaugen einerseits und das Betreiben des Hohlkörpers andererseits aufeinander abgestimmt erfolgen. Auch kann ein den Saugdruck messender Drucksensor datenmäßig mit der Antriebssteuerung verbunden sein, um beispielsweise einen erhöhten Saugdruck als Maß für eine zunehmende Verlegung des Katheterschlauches zu interpretieren und dementsprechend den Antrieb zur Drehung des Hohlkörpers zu steuern.

Die erfindungsgemäße Fördervorrichtung erlaubt das effektive Fördern von Körperflüssigkeit aus dem Körper ohne dass die Gefahr einer zunehmenden Querschnittsreduktion des Katheterschlauches durch koagulierende Substanzen zu befürchten ist. Diese werden nicht nur abgezogen, sondern auch durch Schaben von der Innenumfangsfläche des Katheterschlauches über die gesamte axiale Länge desselben von dessen Innenumfangsfläche entfernt und durch das Saugen an dem Katheterschlauch und/oder den Betrieb eines fördernden Wellenelementes als Hohlkörper, wie beispielsweise einer Helix, aus dem Katheterschlauch herausgeleitet. Der Hohlkörper bewirkt ferner das in der Regel elastische Freistellen des Katheterschlauches, sodass dieser zuverlässig einen Transport durch den Katheterschlauch aufgrund der Höhlung in dem Hohlkörper erlaubt.

Wird beispielsweise ein Patient mit Blut im Herzbeutel mit der erfindungsgemäßen Fördervorrichtung versorgt, so wird zunächst der Katheterschlauch bis in den Herzbeutel geführt. Danach wird der Katheterschlauch auf die gewünschte Länge geschnitten. Danach wird der Hohlkörper in den Katheterschlauch eingeführt. Der Katheterschlauch und der Hohlkörper sind dabei üblicherweise als steril verpackte Versorgungseinheit dem behandelnden Arzt verfügbar gemacht. Nach dem Schneiden des Katheterschlauches hat jedenfalls der Hohlkörper eine größere Länge als der Katheterschlauch. Der Hohlkörper wird bis zu dem an dem distalen Ende vorgesehenen Anschlag in den Katheterschlauch eingeführt.

Danach wird der Hohlkörper mit dem Antriebselement, beispielsweise mit der Hülse drehfest verbunden, wobei zuvor der Katheterschlauchanschluss über das proximale Ende des Katheterschlauches geschoben worden ist.

Sofern erforderlich, wird beispielsweise über eine sich radial erstreckende Feder, beispielsweise in Form einer Madenschraube oder einer Klemmvorrichtung der Hohlkörper mit der Hülse verdrehfest verbunden.

Die Hülse wird sodann in das Kopplungsgehäuse eingebracht. In einer anderen Ausführung ist die Hülse fester Bestandteil des Kopplungsgehäuses, was sich vorteilhaft auf die Dichtheit des Systems auswirkt. Dabei ist die Hülse in der Regel unverlierbar mit dem Kopplungsgehäuse verbunden. Der Katheterschlauchanschluss und der Saugleitungsanschluss werden mit dem Kopplungsgehäuse verschraubt. In dem Kopplungsgehäuse ist dabei üblicherweise eine Antriebshülse vorgesehen, die verdrehfest beispielsweise über eine Nut-Feder-Verbindung mit der Hülse verbunden ist und antriebsmäßig mit einem Antrieb gekoppelt ist. Die Hülse kann ein fester Bestandteil des Kopplungsgehäuses sein.

Der Hohlkörper überragt danach üblicherweise den Saugleitungsanschluss und befindet sich jedenfalls in einer Teillänge der Saugleitung. Diese Saugleitung führt zu einem Auffangbehälter einer Saugvorrichtung, die auch die Saugpumpe, eine Steuerung zu der Saugpumpe und die Antriebssteuerung zu dem Antrieb in sich aufnimmt. Durch Betrieb der Saugpumpe kann Fluid aus dem Herzbeutel abgesaugt werden. Einer drohenden Verstopfung wird durch Betrieb des Antriebs und der Drehung des Hohlkörpers in dem Katheterschlauch vorgebeugt.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
- Fig. 1: eine Seitenansicht wesentlicher Komponenten des Ausführungsbeispiels einer Fördervorrichtung;
- Fig. 2: eine Längsschnittansicht eines Ausführungsbeispiels einer Kopplungseinheit der vorliegenden Erfindung; und
- Fig. 3: eine schematische Darstellung funktioneller Bauteile des Ausführungsbeispiels der Fördervorrichtung.

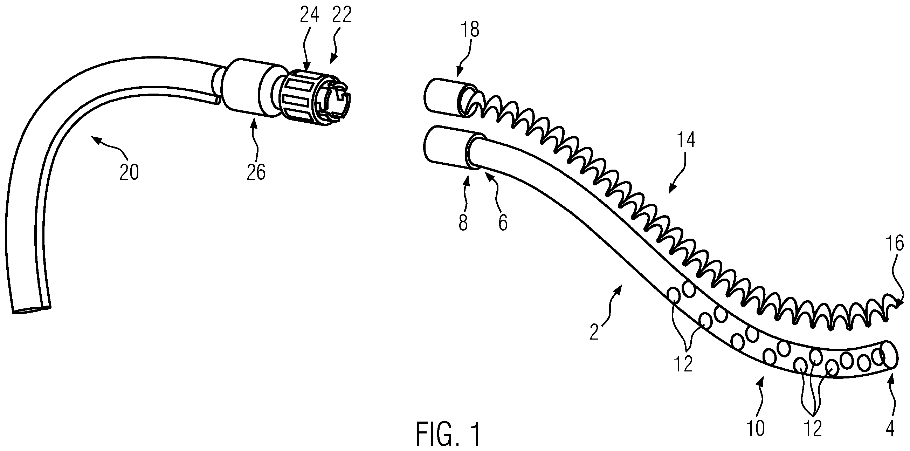

Die Fig. 1 zeigt einen Katheterschlauch 2 mit einem distalen Ende 4 und einem proximalen Ende 6, wobei das proximale Ende 6 mit einem Katheterschlauchanschluss 8 versehen ist. Der mit Bezugszeichen 10 gekennzeichnete distale Endbereich des Katheterschlauches 2 hat an seiner Mantelfläche eine Vielzahl von in Umfangsrichtung und axial beabstandete Öffnungen 12. Mit Bezugszeichen 14 ist ein Hohlkörper gekennzeichnet, der vorliegend als Helix ausgebildet ist, wobei die einzelnen Windungen der Helix zwischen sich einen inneren, sich axial erstreckenden Hohlraum freilassen, der einen Transportkanal 16 ausbildet. Das distale Ende des Hohlkörpers 14 reicht bis zu dem distalen Ende 4 des Katheterschlauches 2.

Das proximale Ende des Hohlkörpers 14 kann in einer Antriebshülse 18 aufgenommen und verdrehfest damit verbunden sein, oder diese Antriebshülse, die ein Beispiel für ein Antriebselement im Sinne der vorliegenden Erfindung ist, überragen.

Zwischen dem Katheterschlauch 2 und einer schlauchförmigen Saugleitung 20 ist an deren Ende ein Kopplungsgehäuse 22 mit daran außenumfänglich befestigten und in Umfangsrichtung wechselnden Magnetfeldern vorgesehen, die einen magnetischen Antrieb 24 ausbilden. Das Kopplungsgehäuse 22 ist zur Aufnahme der Antriebshülse 18 innen hohl. Das Kopplungsgehäuse 22 ist verdrehfest mit der Saugleitung 20 verbunden. Die Antriebshülse 18 trägt außenumfänglich alternierenden Feldern zugeordnete Magneten. Bezugszeichen 26 kennzeichnet einen Saugleitungsanschluss mit einer zylindrischen Manschette zur Aufnahme des magnetischen Antriebs 24.

Die Antriebshülse 18 wird im montierten Zustand von dem Kopplungsgehäuse 22 und dem daran befestigten magnetischen Antrieb 24 umgeben, der durch die umfänglich wechselnden Magnetfelder die Antriebshülse 18 drehend antreibt, welche den Hohlkörper 14 mitnimmt.

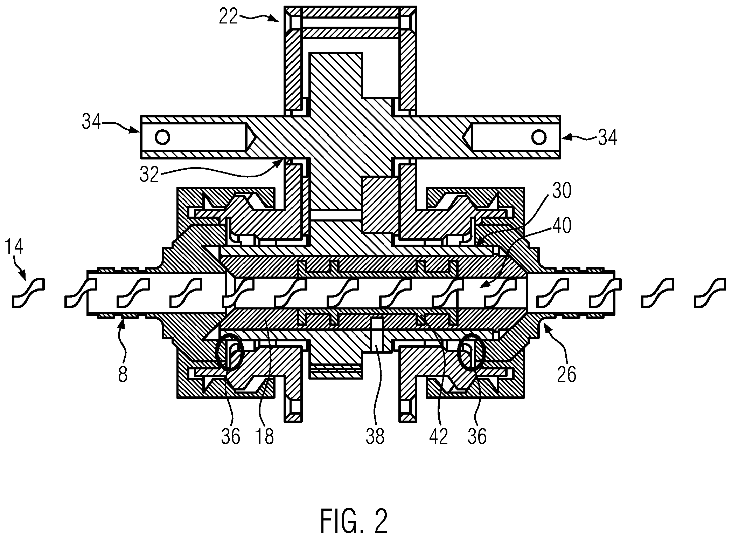

Die Fig. 2 zeigt eine Alternative zum Antrieb des Hohlkörpers 14, wobei auf die Darstellung des Katheterschlauches 2 und der Saugleitung 20 verzichtet wurde. Gleiche Bauteile sind gegenüber dem vorherigen Ausführungsbeispiel mit gleichen Bezugszeichen gekennzeichnet. Das Ausführungsbespiel verdeutlicht indes gegenüber dem Beispiel nach Fig. 1 auch Schlauchanschlüsse für den Katheterschlauch 2 und die Saugleitung 20 detailliert.

Bei dem zweiten Ausführungsbeispiel nach Fig. 2 wird das Kopplungsgehäuse 22 durch einen Kunststoffkörper gebildet, mit dem der Katheteranschluss 8 und Saugleitungsanschluss 26 lösbar verbunden sind. In diesem Ausführungsbeispiel können der Katheterschlauch 2 und die Saugleitung 20 auf den Katheteranschluss 8 bzw. den Saugleitungsanschluss 26 mit deren offenen Enden ohne zusätzlichen Verbindungselementen einfach aufgesteckt werden. Das Kopplungsgehäuse 22 lagert drehbar die Antriebshülse 18 und eine Hülse 30, die verdrehfest mit der Antriebshülse 18 verbunden ist, sowie ein Antriebselement in Form eines Hülsenantriebselementes 32. Das Hülsenantriebselement 32 hat eine Verzahnung, die mit einer Verzahnung an der Hülse 30 kämmt. Das Hülsenantriebselement 32 hat eine Antriebswelle 34, die das Kopplungsgehäuse 22 zur antriebsmäßigen Kopplung mit einem nicht gezeigten Antrieb, beispielsweise in Form eines Elektromotors überragt.

Der Katheteranschluss 8 und der Saugleitungsanschluss 26 sind unter Zwischenlage von Dichtungen 36 mit dem Kopplungsgehäuse 22 verschraubt. Mit Bezugszeichen 38 ist eine Madenschraube angedeutet, die die Antriebshülse 18 durchsetzt und gegen die Hülse 30 anliegt, um diese verdrehfest mit dem Hohlkörper 14 zu verbinden.

Die Fig. 2 lässt einen Durchflusskanal 40 erkennen, der zwischen dem Katheteranschluss 8 und dem Saugleitungsanschluss 26 vorgesehen ist und jedenfalls teilweise durch die Antriebshülse 18 ausgebildet wird. Bei dem gezeigten Ausführungsbeispiel wird innerhalb der Antriebshülse 18 der Durchflusskanal 40 über eine gewisse Teillänge von einer Vorspannhülse 42 definiert, die verdrehfest mit der Antriebshülse 18 verbunden ist. Zwischen der Innenumfangsfläche der Antriebshülse 18 und der Außenumfangsfläche der Vorspannhülse 42 kann ein Vorspannmittel, beispielsweise in Form eines Federelementes oder eines kompressiblen Gases vorgesehen sein, welches die Vorspannhülse 42 radial so vorspannt, dass der durch die Vorspannhülse 42 gebildete Innendurchmesser kleiner als der Außendurchmesser des Hohlkörpers 14 ist. Beim Hindurchführen des Hohlkörpers 14 durch die Vorspannhülse 42 ergibt sich eine radiale Aufweitung derselben, wobei sich gleichzeitig zwangsläufig eine verdrehfeste Verbindung zwischen der Antriebshülse 18 und dem Hohlkörper 14 ergibt. Die zuvor beschriebene Variante stellt eine einfache Möglichkeit dar, den Hohlkörper 14 durch Hindurchführen des Hohlkörpers 14 durch die Antriebshülse 18 auch verdrehfest mit dieser zu verbinden.

Die verdrehfeste Verbindung zwischen der Antriebshülse 18 und der Hülse 30 kann sich auch durch sich in Längsrichtung des Durchflusskanals 40 erstreckende und zwischen diesen beiden angeordneten Nuten und Federn zwangsläufig beim axialen Einschieben der Antriebshülse 18 in die Hülse 30 ergeben. Auf eine Madenschraube 38 kann also auch verzichtet werden.

Die axiale Festlegung der Antriebshülse 18 erfolgt dabei durch die beiden Anschlüsse 8, 26, die die Antriebshülse 18 fluiddicht in dem Kopplungsgehäuse 22 aufnehmen.

Bei dem gezeigten Ausführungsbeispiel überragt die Antriebswelle 34 an gegenüberliegenden Seiten das Kopplungsgehäuse 22, so dass ein Anschluss mit verschiedenen Kopplungen möglich ist.

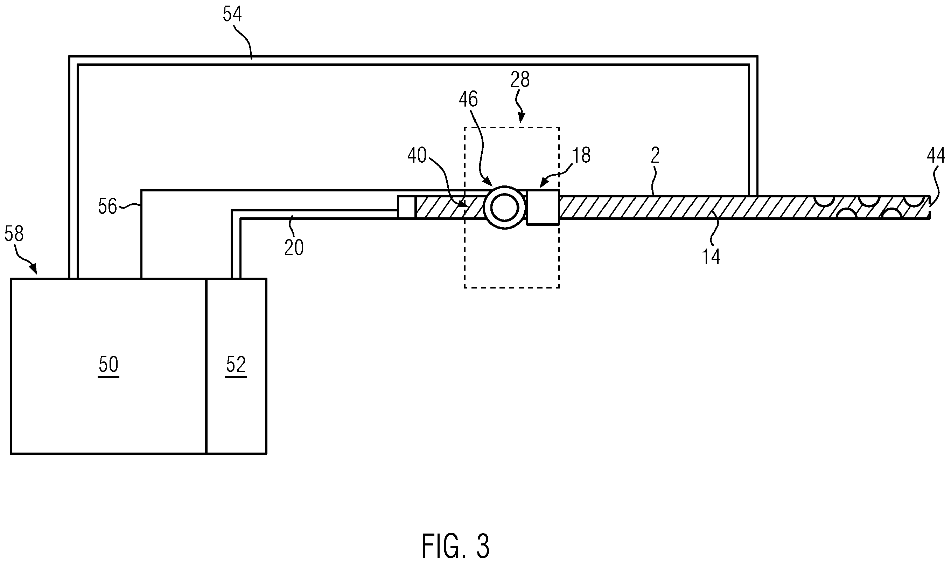

Die Fig. 3 zeigt schematisch weitere Komponenten eines Ausführungsbeispiels. Gleiche Bauteile sind gegenüber den vorherigen Ausführungsbeispielen mit gleichen Bezugszeichen gekennzeichnet.

Die Fig. 3 verdeutlicht den Zustand nach der Versorgung eines Patienten mit dem Katheterschlauch 2, in dem der Hohlkörper 14 vorgesehen ist. Der Hohlkörper 14 erstreckt sich distal bis zu einem mit Bezugszeichen 44 gekennzeichneten axialen Anschlag, der die Einlassöffnung an dem distalen Ende des Katheterschlauches 2 radial verengt, gleichwohl einen Einlass in den Katheterschlauch 2 erlaubt. Ersichtlich erstreckt sich der Hohlkörper 14 bis über die schematisch gekennzeichnete Kopplungseinheit 28 hinaus, die vorliegend neben der Antriebshülse 18 einen Drucksensor 46 in sich aufnimmt, der den Innendruck in dem Katheterschlauch 2 bzw. dem Durchflusskanal 40 oder einer Saugleitung 20 erfasst. Jedenfalls ist das Signal des Drucksensors 46 indikativ für den Saugdruck innerhalb des Katheterschlauches 2. Dieser Drucksensor 46 kann datenmäßig mit einer Steuervorrichtung 50 kommunizieren, die sowohl den Antrieb der Kopplungseinheit 28 wie auch eine Saugpumpe steuert, die üblicherweise in baulicher Einheit mit der Steuervorrichtung 50 vorgesehen ist und einen Unterdruck in der Saugleitung 20 erzeugt, durch welche die über den Katheterschlauch 2 abgesaugte Flüssigkeit in einen Auffangbehälter 52 gelangt.

Die Steuervorrichtung 50 steuert dabei die Funktion der Saugpumpe wie auch das Steuern eines Antriebs, welcher den Hohlkörper 14 antreibt.

Mit Bezugszeichenzeichen 54 ist eine Spülleitung gekennzeichnet, mit der abhängig von Signalen der Steuervorrichtung 50 die Saugleitung 20 gespült werden kann. Die Spülleitung 54 kann in einer alternativen Ausführung auch so positioniert sein, dass der fluidführende Bereich der Kopplungseinheit 28 oder der Katheterschlauch 2 zumindest teilweise gespült werden kann.

Bezugszeichen 56 deutet eine biegbare Antriebswelle an, die einen nicht gezeigten Antrieb innerhalb einer medizinal-technischen Saugvorrichtung 58, die die Steuervorrichtung 50 in sich aufnimmt und üblicherweise den Auffangbehälter 52 lösbar hält, mit der Antriebswelle 34 verbindet.

### Bezugszeichenliste

- 2: Katheterschlauch
- 4: distales Ende
- 6: proximales Ende
- 8: Katheterschlauchanschluss
- 10: distaler Endbereich
- 12: Öffnungen
- 14: Hohlkörper
- 16: Transportkanal
- 18: Antriebselement - Antriebshülse
- 20: Saugleitung
- 22: Kopplungsgehäuse
- 24: magnetischer Antrieb
- 26: Saugleitungsanschluss
- 28: Kopplungseinheit
- 30: Hülse
- 32: Antriebselement- Hülsenantriebselement
- 34: Antriebswelle
- 36: Dichtungen
- 38: Madenschraube
- 40: Durchflusskanal
- 42: Vorspannhülse
- 44: Anschlag
- 46: Drucksensor
- 50: Steuervorrichtung
- 52: Auffangbehälter
- 54: Spülleitung
- 56: Antriebswelle
- 58: Saugvorrichtung

## Patentansprüche

1. Fördervorrichtung für Körperflüssigkeit mit einem Katheterschlauch (2), der ein in den Körper einzuführendes distales Ende (4) und ein proximales Ende (6) hat, **gekennzeichnet durch** einen Hohlkörper (14), der längsverschieblich und/oder drehbar in dem Katheterschlauch (2) angeordnet ist, sich von dem distalen Ende (4) zumindest bis zu dem proximalen Ende (6) erstreckt und über seine Länge einen sich in Erstreckungsrichtung des Katheterschlauches (2) erstreckenden Transportkanal (16) ausbildet.

2. Fördervorrichtung nach Anspruch 1, **dadurch gekennzeichnet, dass** der Hohlkörper (14) über seine Länge mit mindestens einem sich radial erstreckenden Schabsegment versehen ist.

3. Fördervorrichtung nach Anspruch 1 oder 2, **dadurch gekennzeichnet, dass** der Hohlkörper (14) durch eine Helix gebildet ist.

4. Fördervorrichtung nach einem der vorherigen Ansprüche, **dadurch gekennzeichnet, dass** der Katheterschlauch (2) im Bereich seines distalen Endes (4) an seiner Mantelfläche zumindest eine Öffnung (12) aufweist und dass der Hohlkörper (14) eine mit der Öffnung (12) zusammenwirkende Schabkante ausbildet.

5. Fördervorrichtung nach einem der vorherigen Ansprüche, **dadurch gekennzeichnet, dass** der Katheterschlauch (2) an seinem distalen Ende einen axialen Anschlag (44) für den Hohlkörper (14) aufweist.

6. Fördervorrichtung nach einem der vorherigen Ansprüche, **dadurch gekennzeichnet, dass** der Hohlkörper (14) in ein Antriebselement (18) einbringbar und mit dem Antriebselement koppelbar ist.

7. Fördervorrichtung nach einem der vorherigen Ansprüche **gekennzeichnet durch** eine Kopplungseinheit (28) mit einem Kopplungsgehäuse (22), das einen Katheterschlauchanschluss (8) und einen Saugleitungsanschluss (26) aufweist, die mit einem von dem Kopplungsgehäuse (22) umgebenen Durchflusskanal (40) kommunizieren.

8. Fördervorrichtung nach Anspruch 7 **gekennzeichnet durch** ein drehbar an dem Kopplungsgehäuse (22) gelagertes Antriebselement (18, 30, 32) zum Antrieb des Hohlkörpers (14).

9. Fördervorrichtung nach Anspruch 7 oder 8, **dadurch gekennzeichnet, dass** der Saugleitungsanschluss (26), der Katheterschlauchanschluss (8) und/oder das Antriebselement (18) einen zum Durchführen des Hohlkörpers (14) angepasst ausgebildeten Innendurchmesser aufweist.

10. Fördervorrichtung nach einem der Ansprüche 7 bis 9, **dadurch gekennzeichnet, dass** der Durchflusskanal (40) zumindest teilweise durch eine Hülse (18, 30) gebildet ist, die drehbar in dem Kopplungsgehäuse (22) gelagert und lösbar verdrehfest mit dem Hohlkörper (14) verbindbar ist.

11. Fördervorrichtung nach Anspruch 10, **dadurch gekennzeichnet, dass** die Hülse (30) lösbar mit dem Kopplungsgehäuse (22) und lösbar antriebsmäßig mit einem Hülsenantriebselement (32) verbindbar ist.

12. Fördervorrichtung nach einem der Ansprüche 6 bis 11, **dadurch gekennzeichnet, dass** das Hülsenantriebselement (32) eine an dem Kopplungsgehäuse (22) freiliegende Antriebswelle (34) aufweist.

13. Fördervorrichtung nach einem der vorherigen Ansprüche **gekennzeichnet durch** eine mit dem Katheterschlauch (2) und/oder mit der Saugleitung (20) und/oder mit dem Durchflusskanal (40) kommunizierende Spülleitung (54).

14. Fördervorrichtung nach einem der Ansprüche 6 bis 13 **gekennzeichnet durch** einen Antrieb (24), der antriebmäßig mit dem Antriebselement (18) koppelbar ist, und eine dem Antrieb (24) zugeordnete Antriebssteuerung, die derart ausgebildet ist, dass der Hohlkörper (14) über den Antrieb (24) kontinuierlich und/oder reversierend und/oder zyklisch drehend antreibbar ist.

15. Fördervorrichtung nach Anspruch 14, **dadurch gekennzeichnet, dass** die Antriebssteuerung (50) eine Drehmomenterkennung aufweist und dass das erkannte Drehmoments in die Steuerung des Antriebs (24) eingeht.

16. Fördervorrichtung nach Anspruch 14 oder 15, **dadurch gekennzeichnet, dass** die Antriebssteuerung (50) in baulicher Einheit mit einer Saugsteuerung einer Saugpumpe verwirklicht ist, die einem Auffangbehälter (52) zugeordnet ist, der mit der Saugleistung (48) verbindbar ist.

17. Fördervorrichtung nach einem der Ansprüche 14 bis 16, **dadurch gekennzeichnet, dass** die Antriebsteuerung (50) datenmäßig mit einem einen Saugdruck in der Saugleitung messenden Drucksensor (46) verbunden ist.
